(19) Europäisches Patentamt
European Patent Office
Office européen des brevets

(11) Veröffentlichungsnummer: **0 194 591**
**B1**

(12) # EUROPÄISCHE PATENTSCHRIFT

(45) Veröffentlichungstag der Patentschrift: **24.08.88**

(51) Int. Cl.⁴: **C 07 C 45/73**, C 07 C 49/395

(21) Anmeldenummer: **86102996.5**

(22) Anmeldetag: **06.03.86**

(54) **Verfahren zur Herstellung von 2-Alkyl-cyclopentanonen.**

(30) Priorität: **09.03.85 DE 3508420**

(43) Veröffentlichungstag der Anmeldung:
**17.09.86 Patentblatt 86/38**

(45) Bekanntmachung des Hinweises auf die Patenterteilung:
**24.08.88 Patentblatt 88/34**

(84) Benannte Vertragsstaaten:
**BE CH DE FR GB IT LI NL**

(56) Entgegenhaltungen:
**EP - A - 0 013 385**
**EP - A - 0 028 703**
**EP - A - 0 100 019**
**EP - A - 0 127 835**
**FR - A - 2 056 450**
**US - A - 3 829 495**
**US - A - 4 146 581**

(73) Patentinhaber: **BASF Aktiengesellschaft, Carl-Bosch-Strasse 38, D-6700 Ludwigshafen (DE)**

(72) Erfinder: **Gramlich, Walter, Dr., Auf der Hoehe 11, D-6803 Edingen-Neckarhausen (DE)**
Erfinder: **Lengsfeld, Wolfgang, Dr., Woogstrasse 46, D-6703 Limburgerhof (DE)**

**EP 0 194 591 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein gegenüber dem Stand der Technik wesentlich verbessertes Verfahren zur Herstellung von in α-Stellung monoalkylierten Cyclopentanonen.

Die Einführung von Alkylgruppen in α-Stellung des Cyclopentanon-Grundgerüstes ist an sich bekannt.

So wird beispielsweise von M. Hajak et. al. (vgl. Synthesis 1976, Seiten 315 bis 318) ein Syntheseweg beschritten, bei dem Cyclopentanon mit 1-Alkenen, wie 1-Octen, in Gegenwart von Blei(IV)oxid, Mangan-(IV)-oxid oder Kupferoxid und geringen Mengen an Essigsäure zu α-alkylierten Cyclopentanonen wie 2-Octyl-cyclopentanon umgesetzt wird. Die bei diesem Verfahren erzielten Ausbeuten schwankten laut Angaben der Autoren zwischen 64 und 77%.

Nach einem prinzipiell gleichen Verfahren erhielten S. D. Mekhtiev et. al. (vgl. Azerb. Neft. Khoz. 9 (1975), Seiten 69 bis 79) bei der Umsetzung von Cyclopentanon mit 1-Hepten, trotz Optimierung das 2-Heptylcyclopentanon nur in Ausbeuten von 53% der Theorie.

Weiterhin ist die Alkylierung von Cyclopentanon mit Alkylhalogeniden in Gegenwart von Basen bekannt. So beschrieben Gupta et. al. (vgl. J. Indian Chem. Soc. 30 (1953), Seiten 23 bis 25) die Alkylierung von Cyclopentanon mit Alkylbromiden, z.B. Heptylbromid, in Gegenwart von Natriumamid. Hierbei konnte jedoch das erhaltene 2-Heptyl-cyclopentanon infolge von zahlreichen Nebenprodukten nicht rein isoliert werden.

Das Problem einer Mehrfachalkylierung umging K. Takahashi (vgl. Chem. Pharm. Bull. 20 (1972), Seiten 197 bis 201) dadurch, dass er das Cyclopentanon zunächst mit Morpholin in Gegenwart von p-Toluolsulfonsäure in Benzol als Lösungsmittel durch Entfernung des Reaktionswassers in das Enamin überführte, welches dann mit Alkenylbromiden in 46 bis 59%igen Ausbeuten zu den entsprechenden alkylencyclopentanonen umgesetzt wurde. In einer dritten Stufe mussten dann die ungesättigten Verbindungen in Gegenwart eines Katalysators zu den Alkylcyclopentanonen hydriert werden. Nachteilig an diesem Verfahren ist, dass drei Verfahrensschritte benötigt werden und die Ausbeuten zu wünschen übrig lassen.

Auch bei dem Verfahren von A. Jjima und K. Takahashi (vgl. Chem. Pharm. Bull. 21 (1973), S. 215 bis 219) ging man den Umweg über das aus Cyclopentanon und Morpholin erhältliche Enamin.

Hierbei mussten Cyclopentanon und Morpholin in Gegenwart von p-Toluolsulfonsäure in Benzol zunächst 12 Stunden zum Sieden erhitzt werden, um das Reaktionswasser abzutrennen. Danach wurde mit verdünnter Salzsäure eine Stunde gerührt, anschliessend wurden die Phasen getrennt, die wässrige Phase noch einmal mit Benzol extrahiert, dann die vereinigten Benzolphasen mit 5%iger $NaHCO_3$-Lösung und Wasser gewaschen, das Benzol abdestilliert und der Rückstand fraktioniert.

Das erhaltene Enamin wurde in einer zweiten Stufe mit einem Aldehyd erneut 12 Stunden in Benzol als Lösungsmittel unter Rückfluss zum Sieden erhitzt. Die abgekühlte Lösung wurde dann mit verdünnter Salzsäure angesäuert, 1 Stunde lang gerührt, mit Benzol extrahiert, der benzolische Extrakt mit Wasser gewaschen, das gesamte Benzol abdestilliert und der Rückstand fraktioniert. Die Alkylidencyclopentanone wurden so in einer Ausbeute von 40 bis 75% erhalten.

In einer dritten Stufe wurden die Alkylidenverbindungen dann in Gegenwart eines Hydrierkatalysators zu den α-Alkyl-cyclopentanonen hydriert. Nachteilig an diesem Verfahren ist, dass es aus drei zum Teil recht langwierigen Verfahrensschritten besteht sowie dass es nur mittlere Ausbeuten ergibt. Ausserdem ist ein Herstellungsverfahren unter Mitverwendung von Aminen schon deshalb nicht sehr empfehlenswert, da die alkylierten Cyclopentanone zum grossen Teil als Riechstoffe verwendet werden und da deshalb an ihre olfaktorischen Eigenschaften höchste Ansprüche gestellt werden.

Weiterhin ist die direkte Umsetzung von Cyclopentanon mit Aldehyden in Gegenwart von basischen Katalysatoren bekannt (vgl. DRP 633 595 von 1933).

Die nach diesem Verfahren erzielten Ausbeuten liegen zwischen 23,3 und 45,2%. Als hauptsächliche Nebenprodukte traten — wie schon u.a. von Vorländer in Chem. Ber. 29 (1896), S. 1836 lange vorher beschrieben — die mit zwei Molekülen Aldehyd kondensierten Cyclopentanone auf. Bei der anschliessenden katalytischen Hydrierung wurden dann zwar Ausbeuten von 90 bis 95% an α-Alkyl-cyclopentanonen erzielt, jedoch kann bei dem Gesamtverfahren nicht von einem wirtschaftlichen Verfahren die Rede sein.

Es war daher die Aufgabe der Erfindung, ein wirtschaftlicheres Verfahren für die Herstellung der als Riechstoffe begehrten 2-Alkyl-cyclopentanone zu finden, das die Nachteile der bekannten Verfahren nicht aufweist, d.h. mit dessen Hilfe man die 2-Alkylcyclopentanone in einem einfachen einstufigen Verfahren mit guten Ausbeuten herstellen kann.

Es wurde nun überraschenderweise gefunden, dass sich in α-Stellung zur Ketogruppe unsubstituierte Cyclopentanone mit vielen aliphatischen Aldehyden unter praktisch vollständigem Umsatz in hoher Selektivität in einer Einstufenreaktion zu den gewünschten α-monoalkylierten Cyclopentanonen umsetzen lassen, wenn man die Reaktionspartner bei Temperaturen zwischen 80 und 280°C in Gegenwart von Wasserstoff in einem Autoklaven an einem Katalysator umsetzt, der eine Komponente zur Katalytischen Kondensation und eine zweite Komponente zur katalytischen Hydrierung enthält.

Gegenstand der Erfindung ist dementsprechend ein Verfahren zur Herstellung von in 2-Stellung monoalkylierten Cyclopentanonen der allgemeinen Formel I

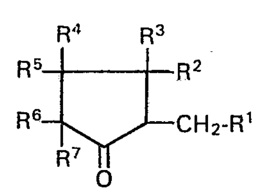

(I)

in der

$R^1$ für eine verzweigte oder unverzweigte aliphatische Gruppen mit 2 bis 12, vorzugsweise 4 bis 8 C-Atomen, bedeutet

und die Reste $R^2$ bis $R^7$ gleich oder verschieden sein können und für Wasserstoff oder einen Alkylrest mit 1 bis 5 C-Atomen, vorzugsweise für Wasserstoff oder eine Methylgruppe stehen, das dadurch gekennzeichnet ist, dass man ein Cyclopentanon der allgemeinen Formel II

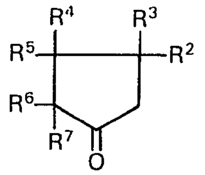

(II)

oder ein entsprechendes Cyclopentanon mit einem Aldehyd der allgemeinen Formel III

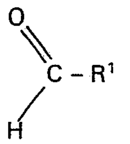

(III)

in der $R^1$ die oben angegebene Bedeutung hat, bei Temperaturen zwischen 80 und 280°C, vorzugsweise zwischen 100 und 200°C in Gegenwart von Wasserstoff in einem Autoklaven an einem Katalysator umsetzt, der als wirksame Bestandteile einerseits ein Oxid oder ein Phosphat des Magnesiums, Aluminiums, Titans, Zinks oder eines der Seltenen Erdmetalle und andererseits ein Edelmetall der Gruppe VIII des Periodensystems, vorzugsweise Palladium, enthält.

Mit besonderem Vorteil gelingt das Verfahren wenn man die Umsetzung unter einem Wasserstoffdruck von 1 bis etwa 300, vorzugsweise 10 bis etwa 100 bar, insbesondere 10 bis 50 bar durchführt.

Sehr vorteilhaft gestaltet sich das Verfahren, wenn man die Umsetzung in Abwesenheit wesentlicher Mengen eines Lösungsmittels durchführt, da hierdurch ohne Ausbeuteverlust die Raum-Zeit-Ausbeuten wesentlich verbessert und die Aufarbeitung erleichtert werden.

Es waren zwar aus den Patentschriften DE 2 615 308, und US-A-4 146 581 schon Verfahren bekannt, mit deren Hilfe man Aldehyde und Ketone in Gegenwart von $H_2$ und Oxiden oder Salzen der Seltenen Erdmetalle und Metallen der Gruppe VIII des Periodensystems der Elemente zu höheren Ketonen umsetzen konnte, jedoch wurden in beiden Verfahren ausschliesslich Ketone eingesetzt, die mindestens eine Methylgruppe in Nachbarstellung zur Carbonylgruppe enthielten. In der US-PS 4 146 581 wird zudem ausgeführt, dass in Ketonen, die in α-Stellung zur Carbonylgruppe eine Methylgruppe und eine Methylengruppe tragen, der Angriff des Aldehyds nahezu ausschliesslich an der Methylgruppe erfolgt.

Auch gemäss dem Verfahren der EP-A-1-0 127 835, gemäss dem höhere gesättigte Ketone durch Umsetzen von bestimmten α,β-ungesättigten Aldehyden mit Ketonen in Gegenwart von $H_2$ und Oxiden oder Phosphaten der Seltenen Erdmetalle, des Magnesiums, Aluminiums, Zinks oder Titans und Metallen der Gruppe VIII des Periodensystems hergestellt werden, werden ausschliesslich Ketone, die eine Methylgruppe in α-Stellung zur Carbonylgruppe tragen eingesetzt. Der Angriff des Aldehyds erfolgte im wesentlichen an der Methylgruppe. Der Fachmann musste daher erwarten, dass bei Ketonen, die keine Methylgruppe enthalten, der Angriff des Aldehyds wesentlich erschwert ist, so dass Selbstkondensationen des Aldehyds oder des Ketons eintreten, bevor eine Kondensation an der (bzw. den) Methylengruppe(n) des Cyclopentanons stattfinden kann. Falls eine Alkylierung des Cyclopentanons überhaupt eintreten würde, waren unerwünschte Mehrfachalkylierungen zu erwarten.

Demgegenüber werden die hohen Selektivitäten des neuen Verfahrens selbst dann erzielt, wenn die Reste $R^6$ und $R^7$ gleich Wasserstoff sind, d.h. wenn α,α'-unsubstituierte Cyclopentanone eingesetzt werden.

Die erzielten Selektivitäten sind insofern überraschend, als unter den angewandten Reaktionsbedingungen sowohl Eigenkondensation des Cyclopentanons, als auch Eigenkondensation der eingesetzten Aldekyde und Mehrfach-Heteroaldolkondensationen (1 Molekül 2-Alkyl-cyclopentanon mit einem weiteren Molekül Aldehyd zu 2,5-Dialkyl-cyclopentanonen) hätten eintreten können.

Wie aus den folgenden Beispielen hervorgeht, finden bei der erfindungsgemässen Umsetzung die oben erwähnten Nebenreaktionen praktisch nicht statt.

Das erfindungsgemässe Verfahren wird in einem Autoklaven im allgemeinen derart durchgeführt, dass man alle Komponenten, d.h. das Cyclopentanon der Formel II oder das entsprechende Cyclopentanon, den Aldehyd der Formel III und den heterogenen Katalysator vorlegt, den Autoklaven verschliesst und dann zunächst mit Stickstoff und danach mit Wasserstoff spült. Anschliessend wird dann unter Aufpressen von Wasserstoff auf die Reaktionstemperatur erhitzt.

Mit besonderem Vorteil arbeitet man so, dass man in den Autoklaven nach dem Spülen mit Wasserstoff einen Wasserstoffdruck von 2 bis 20 bar aufpresst, anschliessend die Temperatur erhöht und bei Erreichen der Umsetzungstemperatur den Wasserstoffdruck auf 40 bis 50 bar erhöht.

Hierbei finden sowohl die Aldolkondensation als auch die anschliessende Hydrierung in einem Schritt statt, wobei der verbrauchte Wasserstoff laufend nachgepresst wird.

Nach Abschluss der Reaktion wird der Autoklav abgekühlt, entspannt und der Austrag über Filter herausgesaugt und so vom Katalysator abgetrennt. Durch die gleichen Filter kann der Katalysator für den nächsten Ansatz mit neuen Ausgangsstoffen in den Autoklaven zurückgespült werden. Alternativ kann man den Katalysator auch gleich im Autoklaven belassen, indem man ein entsprechend langes Steigrohr mit Fritte zum Austragen der flüssigen Phase benutzt. Der Katalysator lasst sich mehrfach einsetzen. Der erhaltene flüssige Reaktoraustrag braucht nur

aufdestilliert zu werden. Gegebenanfalls kann man das entstandene Reaktionswasser vor der Destillation als untere Phase abtrennen.

Diese beschriebene sogenannte Batch-Fahrweise ist für Umsetzungen im kleineren technischen Massstab geeignet. Für eine grosstechnische Herstellung kann man den Katalysator jedoch auch als Trägerkatalysator, z.B. in Form von Kugeln oder Strängen, in eine Reaktorsäule fest einbauen und das Gemisch aus Cyclopentanon oder -pentenon und Aldehyd unter den erfindungsgemässen Bedingungen kontinuierlich über den Katalysator führen.

Der erfindungsgemässe Katalysator stellt — wie bereits oben erwähnt — ein Katalysatorsystem aus prinzipiell zwei Komponenten dar.

Die eine Komponente dient dem Kondensationsschritt. Geeignet für diesen Schritt sind die Oxide oder Phosphate des Magnesiums, Aluminiums, Titans, Zinks sowie eines der seltenen Erden, wie Praseodymoxid ($Pr_4O_6$). Als besonders geeignet und vorteilhaft haben sich die Oxide des Aluminiums erwiesen.

Als zweite Komponente enthält der Katalysator ein Edelmetall der VIII. Gruppe des Periodensystems, insbesondere Palladium.

Die übrigen Edelmetalle — Platin, Ruthenium, Rhodium, Iridium und Osmium — sind ebenfalls geeignet, kommen aber aus wirtschaftlichen Gründen in der Regel weniger in Betracht.

Der Katalysator kann sowohl in Form eines Gemisches aus dem Kondensationskatalysator, wie z.B. $Al_2O_3$, und dem Hydrierkatalysator, wie Palladium auf Aktivkohle, als auch in Form eines einheitlichen Katalysators, beispielsweise einem der Palladium auf $Al_2O_3$ enthält, vorliegen.

Wesentlich ist nur die gleichzeitige Anwesenheit beider Katalysator-Komponenten.

Der Gehalt an Edelmetall liegt im allgemeinen zwischen 0,1 und 5 Gew.-%. Bevorzugt werden Katalysatoren, die Palladium als Edelmetall mit einem Gehalt von 0,5 bis 1%, bezogen auf die eingesetzte Katalysatormenge enhalten.

Bei kontinuierlicher Fahrweise werden Suspensionskatalysatoren eingesetzt. Führt man die Reaktion kontinuierlich in einer Reaktorsäule durch, so verwendet man vorzugsweise Trägerkatalysatoren, auf welche das Edelmetall, z.B. das Palladium aufgebracht wird. Dabei kann man das Trägermaterial, das aus der Kondensationskomponente besteht, mit einer wässrigen Lösung, die ein Palladiumsalz wie Palladiumnitrat enthält, imprägnieren.

Der Palladium-Metall bildet sich dann unter den hydrierenden Bedingungen von selber, jedoch kann man auch den Katalysator zu diesem Zwecke einer gesonderten Hydrierung unterwerfen. Der bei kontinuierlicher Fahrweise eingesetzte Katalysator kann in Form von Tabletten, Granulaten, Kugeln oder Strängen eingesetzt werden, bei diskontinuierlicher Fahrweise verwendet man häufig die Pulverform.

Als Cyclopentanone der allgemeinen Formel II kommen beispielsweise in Betracht:
Cyclopentanon, 3-Methyl-cyclopentanon, 2-Methyl-cyclopentanon, 3,4-Dimethyl-cyclopentanon, 2,4-Dimethyl-cyclopentanon, 2,3-Dimethyl-cyclopentanon, 2,3,4-Trimethyl-cyclopentanon, 2,2,4-Trimethyl-cyclopentanon, 2,2,3-Trimethyl-cyclopentanon und 2,2,4-Trimethyl-cyclopentanon. Bevorzugt wird Cyclopentanon eingesetzt, da dessen erfindungsgemäss erhältliche Derivate besonders grosse Bedeutung haben.

Es lassen sich aber auch die entsprechenden Cyclopentanone, wie das 3-Methyl-cyclopent-2-enon einsetzen, wobei auch die vorhandene Doppelbindung, unter den Reaktionsbedingungen, wie die bei der Kondensation entstehende Doppelbindung hydriert wird. Die Doppelbindung kann sich in beliebiger Stellung befinden; in diesem Fall bedeuten $R^2$ bzw. $R^2$ und $R^4$ bzw. $R^4$ und $R^6$ den Anteil einer Doppelbindung.

Als Aldehyde der Formel III lassen sich sowohl die geradkettigen unsubstituierten Aldehyde wie Propanal, Butanal, Pentanal, Hexanal, Heptanal, Octanal, Nonanal, Decanal, Undecanal und Dodecanal als auch verschiedene verzweigte und substituierte einsetzen, z.B. das 2-Methylpropanal, 2-Methyl-butanal, 2-Methyl-pentanal, 2-Methyl-hexanal, 2-Methyl-heptanal, 2-Methyl-octanal, 2-Methyl-nonanal, 2-Methyl-decanal, 2-Methyl-undecanal, 3-Methylbutanal und 3-Methyl-pentanal einsetzen.

Das Mol-Verhältnis von Cyclopentanon zu Aldehyd kann bei 1 zu 1 liegen, bevorzugt wird jedoch ein Überschuss des billigeren Reaktionspartners verwendet, wodurch sich die Selektivität, bezogen auf den teureren Reaktionspartner noch erheblich erhöhen lässt.

Vorteilhaft ist es, die Reaktion ohne Lösungsmittel durchzuführen. Man kann jedoch auch ein Lösungsmittel wie Methanol, Ethanol oder Propanol einsetzen.

Die Reaktionstemperatur für das erfindungsgemässe Verfahren hängt von den Ausgangskomponenten sowie der Art und Menge des eingesetzten Katalysators ab. Sie kann zwischen 80 und 280°C schwanken; bei Verwendung von hoher Katalysatormengen kann sie auch unter 80°C liegen. Der bevorzugte Temperaturbereich für ein wirtschaftliches Verfahren liegt zwischen 100 und 200°C. Die dabei eingesetzte Menge an Katalysator variiert je nach Reaktivität von Aldehyd und Cyclopentanon-Derivat. Vorzugsweise werden Mengen von 3 bis 8 Gew.-%, bezogen auf die Summe der Ausgangsverbindungen verwendet.

Auch der Wasserstoffdruck kann in weiten Grenzen variiert werden und ist abhängig vom Gehalt des eingesetzten Edelmetalles. Bei Verwendung eines häufig eingesetzten Katalysators, der 0,5 Gew.-% Palladium auf Aluminiumoxid enthält (siehe Beispiel 1) sind bei Einsatz von 5 Gew.-% an Katalysator Wasserstoffdrücke zwischen 40 und 50 bar ausreichend. Bei Benutzung eines Katalysators mit einem höheren Palladiumgehalt bzw. bei Anwendung von grösseren Mengen des obigen 0,5% $Pd/Al_2O_3$-Katalysators genügen geringere Wasserstoffdrücke.

Mit Hilfe des erfindungsgemässen Verfahrens gelingt es in α-Stellung zur Ketogruppe unsubstituierte Cyclopentanone oder die entsprechenden Cyclopentenone mit vielen aliphatischen Aldehyden in einer einfachen Einstufenreaktion unter praktisch vollständigem Umsatz in hoher Selektivität zu den begehrten α-monoalkylierten Cyclopentanonen umzu-

setzen. Das erfindungsgemässe Verfahren zeichnet sich aber nicht nur durch bessere Ausbeuten und bessere Raum/Zeit-Ausbeuten aus, sondern zusätzlich durch seine Umweltfreundlicheit, da ohne Hilfsbasen oder Salze nur in Gegenwart eines heterogenen Katalysators gearbeitet wird, und somit keine salzhaltigen Abwässer anfallen.

Ein technisch besonders bedeutendes Produkt des neuen erfindungsgemässen Verfahrens ist 2-Heptylcyclopentanon, das ein wichtiger Jasminriechstoff ist. Aber auch eine Vielzahl weiterer 2-substituierter Cyclopentanone ist als Riechstoff geeignet (vgl. Perfumer and Flavorist 8 (1983). Heft April/Mai, Seiten 68 bis 74).

*Beispiel 1*

Herstellung von 2-Heptyl-cyclopentanon

In einem Autoklaven wurde eine Mischung aus 1000 ml (930 g; 11 mol) Cyclopentanon, 500 ml (425 g, 3,7 mol) n-Heptanal und 70 g eines 0,5 Gew.-% Palladium auf Aluminiumoxid enthaltenden pulverförmigen Katalysators vorgelegt. Nach Spülen mit Stickstoff und Wasserstoff wurde bei 25°C 10 bar Wasserstoffdruck aufgepresst und danach 3 Stunden (h) bei einem Wasserstoffdruck von 35 bar auf 110°C und schliesslich bei einem Wasserstoffdruck von 50 bar bis zur Druckkonstanz auf 160°C erhitzt, wozu ca. 10 h erforderlich waren. Nach Abtrennen des Katalysators wurde der Reaktoraustrag direkt fraktioniert.

Nach Abdestillieren des Vorlaufs (605 g Cyclopentanon bei Kp. 130°C und 5 g einer Zwischenfraktion) wurden 633 g (3,48 mol) an reinem 2-Heptyl-cyclopentanon erhalten (Kp. 93°C/1 mbar), was eine Selektivität von 94%, bezogen auf n-Heptanal, bedeutet. Die Selektivität bezüglich Cyclopentanon betrug 92%.

*Beispiel 2*

Herstellung von 2-Hexyl-cyclopentanon

Analog Beispiel 1 wurde in einem Autoklaven eine Mischung aus 1008 g (12 mol) Cyclopentanon, 800 g (8 mol) n-Hexanal und 80 g eines 0,5 Gew.-% Palladium auf $\gamma$-Aluminiumoxid enthaltenden pulverförmigen Katalysators bei 140°C und 70 bar Wasserstoffdruck bis zur Druckkonstanz hydriert (ca. 15 h). Nach Abtrennen des Katalysators wurde der Reaktoraustrag direkt aufdestilliert. 294 g (3,5 mol) Cyclopentanon wurde dabei als Fraktion 1 zurückgewonnen. Als Hauptfraktion erhielt man 1103 g an reinem 2-Hexyl-cyclopentanon (Kp. 84°C/0,35 mbar), was einer Selektivität, bezogen auf n-Hexanal, von 82% entspricht. Erhöhte man wie im Beispiel 1 das Mol-Verhältnis Cyclopentanon zu Hexanol auf 3:1, so stieg die Selektivität auf über 90% wie im Falle des 2-Heptyl-cyclopentanons.

*Beispiel 3*

Herstellung von 2-(2-Methyl-pentyl)-cyclopentanon

In einem 300 ml-Autoklaven wurde eine Mischung aus 126 g (1,5 mol) Cyclopentanon, 50 g (0,5 mol) 2-Methyl-pentanal und 10 g eines 1 Gew.-% Palladium, 94 Gew.-% Aluminiumoxid und 5 Gew.-% Praseodymoxid enthaltenden pulverförmigen Katalysators tors vorgelegt. Nach Spülen mit Stickstoff und Wasserstoff bei 20°C wurden 10 bar $H_2$-Druck aufgepresst und danach das Reaktionsgemisch bei einem Wasserstoffdruck von 30 bar bis zur Druckkonstanz auf 150°C erhitzt. Nach Entfernen des Katalysators wurde der Autoklavenaustrag fraktioniert. Man erhielt 74,9 g an reinem 2-(2-Methylpentyl)-cyclopentanon (Kp. 125°C/20 mbar), was einer Selektivität von 89,2%, bezogen auf 2-Methyl-pentanal, entspricht.

## Patentansprüche

1. Verfahren zur Herstellung von in 2-Stellung monoalkylierten Cyclopentanonen der allgemeinen Formel I

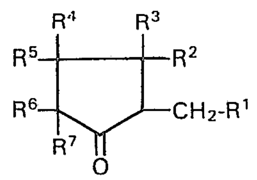

(I)

in der

$R^1$ für eine verzweigte oder unverzweigte aliphatische Gruppe mit 2 bis 12, vorzugsweise 4 bis 8 C-Atomen bedeutet und die Reste $R^2$ bis $R^7$ gleich oder verschieden sein können und für Wasserstoff oder einen Alkylrest mit 1 bis 5 C-Atomen, vorzugsweise für Wasserstoff oder eine Methylgruppe, stehen, dadurch gekennzeichnet dass man ein Cyclopentanon der allgemeinen Formel II

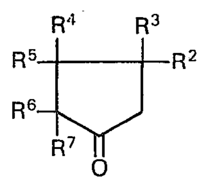

(II)

oder ein entsprechendes Cyclopentanon mit einem Aldehyd der allgemeinen Formel III

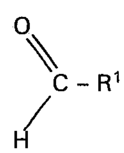

(III)

in der $R^1$ die oben angegebene Bedeutung hat, bei Temperaturen zwischen 80 und 280°C, vorzugsweise zwischen 100 und 200°C in Gegenwart von Wasserstoff in einem Autoklaven an einem Katalysator umsetzt, der als wirksame Bestandteile einerseits ein Oxid oder ein Phosphat des Magnesiums, Aluminiums, Titans, Zinks oder eines der Seltenen Erdmetalle und andererseits ein Edelmetall der Gruppe VIII des Periodensystems, vorzugsweise Palladium, enthält.

2. Verfahren nach Anspruch 1, dadurch gekennzeichnet, dass man die Umsetzung unter einem Wasserstoffdruck von 1 bis etwa 300, vorzugsweise 10 bis 100 bar durchführt.

3. Verfahren nach Anspruch 1, dadurch gekennzeichnet, dass man die Umsetzung in Abwesenheit wesentlicher Mengen eines Lösungsmittels durchführt.

4. Verfahren nach den Ansprüchen 1 bis 3, dadurch gekennzeichnet, dass man ein Gemisch aus den Ausgangsverbindungen und dem Katalysator in dem Autoklaven vorlegt, den Autoklaven nach dem Verschliessen mit einem Inertgas und danach mit Wasserstoff spült, und dann unter Aufpressen von Wasserstoff auf die Reaktionstemperatur erhitzt.

5. Verfahren nach den Ansprüchen 1 bis 4, dadurch gekennzeichnet, dass man in den Autoklaven nach dem Spülen mit Wasserstoff einen Wasserstoffdruck von 2 bis 20 bar aufpresst, anschliessend die Temperatur erhöht und bei Erreichen der Umsetzungstemperatur den Wasserstoffdruck erhöht.

**Claims**

1. A process for the preparation of a cyclopentanone which is monoalkylated in the 2-position and of the formula I

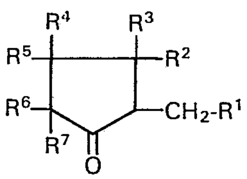

(I)

where $R^1$ is a branched or straight-chain aliphatic group of 2 to 12, preferably 4 to 8, carbon atoms and $R^2$ to $R^7$ may be identical or different and are each hydrogen or alkyl of 1 to 5 carbon atoms, preferably hydrogen or methyl, wherein a cyclopentanone of the formula II

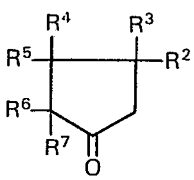

(II)

or a corresponding cyclopentanone, is reacted with an aldehyde of the formula III

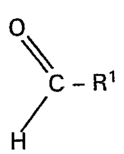

(III)

where $R^1$ has the above meanings, at from 80 to 280°C, preferably from 100 to 200°C, in the presence of hydrogen in an autoclave, over a catalyst which contains, as active components, on the one hand an oxide or a phosphate of magnesium, aluminium, titanium or zinc or of a rare earth metal, and on the other hand a noble metal of group VIII of the periodic table, preferably palladium.

2. A process as claimed in claim 1, wherein the reaction is carried out under a hydrogen pressure of from 1 to about 300, preferably from 10 to 100, bar.

3. A process as claimed in claim 1, wherein the reaction is carried out in the absence of substantial amounts of a solvent.

4. A process as claimed in any of claims 1 to 3, wherein a mixture of the starting compounds and catalyst is initially taken in the autoclave, and the latter is closed, flushed with an inert gas and then with hydrogen, and then heated at the reaction temperature while hydrogen is forced in.

5. A process as claimed in any of claims 1 to 4, wherein the autoclave is flushed with hydrogen, after which hydrogen is forced into the autoclave to a hydrogen pressure of from 2 to 20 bar, the temperature is then increased, and the hydrogen pressure is raised when the reaction temperature is reached.

**Revendications**

1. Procédé de préparation de cyclopentanones monoalkylées en position 2 de formule générale I

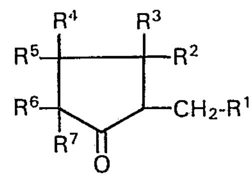

(I)

dans laquelle
$R^1$ est mis pour un groupement aliphatique ramifié ou linéaire ayant de 2 à 12 et de préférence de 4 à 8 atomes de C, et les restes $R^2$ à $R^7$ peuvent être identiques ou linéaires et sont mis pour un atome d'hydrogène ou un reste alkyle de 1 à 5 atomes de C, de préférence pour un atome d'hydrogène ou un groupement méthyle, caractérisé en ce qu'on fait réagir une cyclopentanone de formule générale II

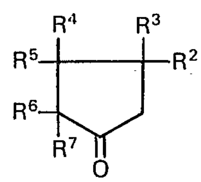

(II)

ou une cyclopentanone correspondante, avec un aldéhyde de formule générale

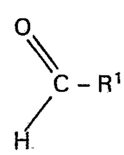

(III)

dans laquelle $R^1$ est tel que défini précédemment, à des températures comprises entre 80° et 280°C, de préférence entre 100° et 200°C, en présence d'hydrogène dans un autoclave, avec un catalyseur qui contient comme composant actif, d'une part un oxyde ou un phosphate de magnésium, d'aluminium, de titane, de zinc ou d'un métal des terres rares et d'autre part un métal noble du groupe VIII de la Classification périodique, de préférence le palladium.

2. Procédé selon la revendication 1, caractérisé en ce qu'on mène la réaction sous une pression d'hydrogène de 1 à environ 300 bars, de préférence de 10 à 100 bars.

3. Procédé selon la revendication 1, caractérisé en ce qu'on mène la réaction en l'absence de quantités importantes d'un solvant.

4. Procédé selon l'une quelconque des revendications 1 à 3, caractérisé en ce qu'on introduit préalablement dans l'autoclave un mélange des composés de départ et du catalyseur, on purge l'autoclave après fermeture avec un gaz inerte puis avec de l'hydrogène, puis on chauffe à la température de réaction sous pression d'hydrogène.

5. Procédé selon l'une quelconque des revendications 1 à 4, caractérisé en ce qu'on établit dans l'autoclave une pression d'hydrogène de 2 à 20 bars après la purge à l'hydrogène, on élève ensuite la température et on élève la pression d'hydrogène lorsque la température de réaction est atteinte.